# EUROPÄISCHE PATENTSCHRIFT

(11) **EP 1 024 771 B1**
(45) Veröffentlichungstag und Bekanntmachung des Hinweises auf die Patenterteilung: **05.06.2002**
(21) Anmeldenummer: 98951506.9
(22) Anmeldetag: 08.10.1998
(51) Int. Cl.: A61F 13/15

(54) **ABSORBIERENDER HYGIENEARTIKEL ZUM EINMALIGEN GEBRAUCH**
DISPOSABLE ABSORBENT HYGIENE ITEM
ARTICLE D'HYGIENE ABSORBANT A USAGE UNIQUE

(30) Priorität: 22.10.1997 DE 19746500
(43) Veröffentlichungstag der Anmeldung: 09.08.2000
(73) Patentinhaber: Paul Hartmann Aktiengesellschaft, 89522 Heidenheim (DE)
(72) Erfinder: MALOWANIEC, Krzysztof, D., D-89522 Heidenheim (DE)
(74) Vertreter: Dreiss, Fuhlendorf, Steimle & Becker
(86) Internationale Anmeldenummer: EP9806408
(87) Internationale Veröffentlichungsnummer: WO9920214

(56) Entgegenhaltungen:
- WO-A-97/33547
- GB-A- 2 216 774
- US-A- 4 964 860

## Beschreibung

Die Erfindung betrifft einen absorbierenden Hygieneartikel zum einmaligen Gebrauch mit den Merkmalen des Oberbegriffs des Anspruchs 1, bekannt bespielsweise aus GB-A-2 216 774.

Im Zuge der fortschreitenden Weiterentwicklung von Hygieneartikeln, wie Windeln, Inkontinenzvorlagen oder - slips, werden die sog. Chassismaterialien, also die zur Bildung des Rückenblatts und den Deckblatts verwendeten Flachbahnmaterialien immer dünner, leichtgewichtiger und damit instabiler. Es werden zunehmend atmungsaktive Folien mit auf das Flächengewicht bezogen geringerer Stabilität bzw. Festigkeit als bei wasserdampfundurchlässigen Folien eingesetzt. Die Projektionsfläche des Saugkörpers senkrecht zur Ebene des Hygieneartikels im flach ausgelegten Zustand wird aufgrund zunehmender Absorptionsfähigkeit des Saugkörpers bzw. der darin enthaltenen, insbesondere superabsorbierende Polymere aufweisenden Materialien immer kleiner. Der Saugkörper mit seinen zumeist bauschelastischen Fasern verleiht dem Hygieneartikel aber eine gewisse Steifigkeit oder Formstabilität. Mit verringerter Größe des Saugkörpers nimmt die Stabilität des Hygieneartikels im Bereich außerhalb des Saugkörpers ab. Daraus ergeben sich Schwierigkeiten bei der Herstellung der Hygienartikel in schnelllaufenden Maschinen; es kommt zu Flattern der Flachbahnmaterialien auf dem Förderband. Darüber hinaus wird die Handhabbarkeit des fertigen Hygieneartikels vom Endverbraucher als unkomfortabel empfunden. Der Hygieneartikel wirkt lapprig. Es besteht Bruch- bzw. Reißgefahr der Chassismaterialien, insbesondere beim Anlegen des Hygieneartikels, da durch die mechanisch wirkenden Verschlusselemente, die üblicherweise auf seitlich abstehenden Verschlusslaschen vorgesehen sind, nachweislich sehr hohe Zugkräfte in die Chassismaterialien eingelaitet werden.

Im Bauchbereich des Hygieneartikels treten diese nachteiligen Wirkungen in geringerem Maße auf, da dort zumeist flächenhaft erstreckte Auftreffelemente für die mechanisch wirkenden Verschlusselemente vorgesehen sind, und zwar in Form von stabilisierenden Frontaltapes mit insbesondere flauschförmiger Oberflächenstruktur, mit der die mechanisch wirkenden Verschlusselemente eine Halteverbindung ausbilden können.

Der vorliegenden Erfindung liegt daher die Aufgabe zugrunde, einen Hygieneartikel der eingangs beschriebenen Art im Rückenbereich zu stabilisieren.

Diese Aufgabe wird bei einem Hygieneartikel der eingangs beschriebenen Art erfindungsgemäß durch die weiteren Merkmale des Anspruchs 1 gelöst.

Mit der Erfindung wurde also erkannt, dass durch Vorsehen der im wesentlichen unelastischen oder zumindest mit einer geringeren Elastizität als die des Rückenblatts versehenen Bandlasche, welche sich im angelegten Zustand des Hygieneartikels gürtelartig vom Rückenbereich in Richtung auf den Bauchbereich erstreckt und zugleich die mechanisch wirkenden Befestigungsmittel bildet, eine Stabilisierung des Rückenbereichs des Hygieneartikels erreicht wird. Die besonders bei mechanisch wirkenden Verschlusselementen auftretenden Kräfte werden nicht mehr lokal in die Chassismaterialien, also Rückenblatt und/oder Deckblatt, eingeleitet, sondern sie werden von der in Umfangsrichtung oder Querrichtung über die gesamte Breite des Rückenbereichs durchgehende Bandlasche aufgenommen und über deren Anbindung an die Chassismaterialien teilweise und gleichmäßig auf diese abgeleitet.

Hinweise auf das Problem oder seine Lösung lassen sich dem Stand der Technik nicht entnehmen. US-A-4,964,860 zeigt einen nicht gattungsgemäßen Hygieneartikel mit einem in Umfangsrichtung geschlossenen wiederverwendbaren Hüftgurt, an dem der Rückenbereich und der Bauchbereich eines einen Saugkörper umfassenden Aufnahmeteils lösbar befestigbar ist. Entsprechendes zeigt die WO-A-97/33547.

GB-A-2 216 774 lehrt, einen vorderen und hinteren Hüftabschnitt zu versteifen, um Wulstbildung ("rollover") zu reduzieren.

Wenn die Bandlasche in vorteilhafter Weise zwischen dem Rückenblatt und dem Deckblatt angeordnet ist, so vermittelt dies einen optisch gefälligen Eindruck und die Zugkräfte werden, wenn überhaupt, auf beide Chassismaterialien übertragen.

Die mechanisch wirkenden Verschlusselemente können in vorteilhafter Weise Teil eines Klettverschluss-Systems sein. Es handelt sich hierbei vorzugsweise um die hakenbildende Komponente des Klettverschluss-Systems.

In ganz besonders vorteilhafter Weiterentwicklung der Erfindung ist die Bandlasche einschließlich der Verschlusselemente einstückig extrudiert. Die Anwendung des Extrusionsverfahrens ist nicht nur kostengünstig, da keine weiteren Verfahrensschritte zum Verbinden einer Bandlasche und zusätzlicher Verschlusselemente erforderlich sind, sondern sie schließt auch die Gefahr des Auftretens schwacher Verbindungsstellen, die dann bevorzugt Bruchstellen bilden, aus.

Bevorzugtermaßen weisen die beiderseitigen Endabschnitte der Bandlasche den jeweiligen Kantenabschluss der Bandlasche bildende Kantenendbereiche auf, die keine mechanischen Verschlusselemente tragen und somit als Greifbereich für die Finger eines Benutzers (Fingerlift) fungieren können.

Nach einem weiteren bevorzugten Erfindungsgedanken weist die Bandlasche einen mittleren Abschnitt mit einer ersten Materialstärke und sich beidseits an den mittleren Abschnitt anschließende Außenabschnitte mit einer zweiten Materialstärke auf, die größer ist als die erste Materialstärke, wobei die Endabschnitte der Bandlasche, welche die mechanischen Verschlusselemente aufweisen, Teil dieser Außenabschnitte der Bandlasche sind. Auf diese Weise kann dort, wo die größten Kräfte auftreten, die Materialstärke entsprechend angepasst werden und in Bereichen geringerer Beanspruchung reduziert werden.

Als noch vorteilhafter wird es angesehen, wenn ein die mechanischen Verschlusselemente tragender Teilbereich der Außenabschnitte eine dritte Materialstärke aufweist, die größer ist als die zweite Materialstärke. Hierdurch sind die mechanisch wirkenden Verschlusselemente, die vorzugsweise die hakenbildende Komponente eines Klettverschluss-Systems bilden, auf einer Art "Podest" angeordnet und können somit effektiver in einen in entsprechender Weise ausgebildeten Auftreffbereich im Bauchbereich des Hygieneartikels eingreifen und damit die Haltefunktion effektiver erfüllen.

Das zumindest abschnittsweise flüssigkeitsdichte Rückenblatt ist vorzugsweise atmungsaktiv; hierunter soll eine Wasserdampfdurchlässigkeit von mindestens 500 g/m² in 24 Stunden nach DIN 35122, Blatt 1 verstanden werden.

Bei einer ganz besonders bevorzugten Ausführungsform des erfindungsgemäßen Hygieneartikels weist die Bandlasche in dem Bereich, in dem sie mit dem Rückenbereich überlappt, eine Vielzahl von durchgehenden Einschnitten auf, die vorzugsweise im wesentlichen linear erstreckt sind. Wenn die Bandlasche an ein Chassismaterial angefügt ist und die Chassismaterialien querelastisch ausgebildet sind, so kann die Bandlasche der elastischen Dehnung der Chassismaterialien folgen, indem sich die Einschnitte beispielsweise rautenförmig öffnen. Die Bandlasche behindert daher die gewolltermaßen elastische Dehnung der Chassismaterialien nicht. In weiterer Ausbildung dieses Erfindungsgedankens erweist es sich als vorteilhaft, wenn die Einschnitte in Längsrichtung des Hygieneartikels erstreckt sind und mehrere Einschnitte in Querrichtung nebeneinander und zur Längsrichtung versetzt zueinander angeordnet sind. Hierdurch wird eine über die Erstreckung der Bandlasche gleichmäßige Wirkung der Einschnitte erreicht.

Weitere Merkmale, Einzelheiten und Vorteile der Erfindung ergeben sich aus den beigefügten Ansprüchen und der zeichnerischen Darstellung und nachfolgenden Beschreibung einer bevorzugten Ausführungsform der Erfindung. In der Zeichnung zeigt:
- Figur 1: eine Draufsicht auf eine bevorzugte Ausführungsform eines erfindungsgemäßen Hygieneartikels in Form einer Windel,
- Figur 2: eine Schnittansicht gesehen in Richtung der Pfeile II-II;
- Figur 3: eine der Figur 2 entsprechende Schnittansicht einer weiteren Ausführungsform einer erfindungsgemäßen Windel; und
- Figur 4: eine Draufsicht auf die Bandlasche nach Figur 3.

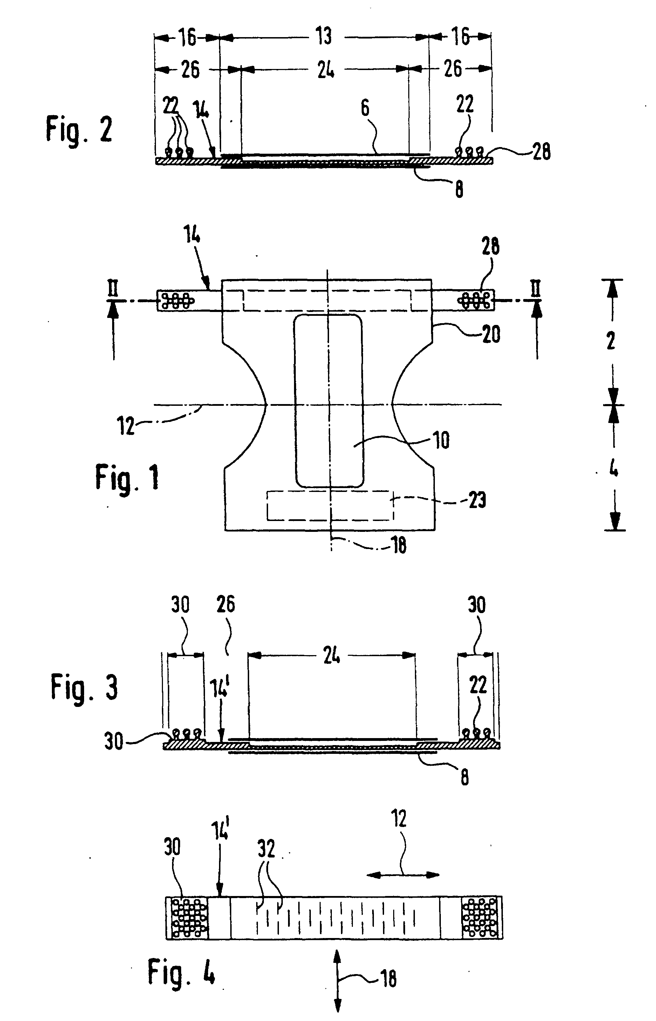

Die Figuren 1 und 2 zeigen einen Hygienartikel in Form einer Windel mit einem Rückenbereich 2 und einem Bauchbereich 4. Die Windel umfasst ein zumindest abschnittsweise flüssigkeitsdurchlässiges Deckblatt 6, ein zumindest abschnittsweise flüssigkeitsdichtes Rückenblatt 8 und einen dazwischen angeordneten Saugkörper 10.

In Querrichtung 12 bzw. parallel zur Querrichtung 12 der Windel erstreckt sich eine über die gesamte Breite 13 des Rückenbereichs 2 durchgehende Bandlasche 14, die wie aus Figur 2 ersichtlich zwischen Deckblatt 6 und Rückenblatt 8 hindurchgehend angeordnet ist. Die Bandlasche 14 steht mit jeweiligen Endabschnitten 16 beidseits über in Längsrichtung 18 verlaufende seitliche Längsränder 20 des chassisbildenden Deckblatts 6 bzw. Rückenblatts 8 vor. Diese Endabschnitte 16 umfassen auch mechanisch wirkende Verschlusselemente 22, bei denen es sich um die hakenbildende Komponente eines Klettverschluss-Systems handelt. Im Bauchbereich 4 der Windel ist auf der Außenseite des Rückenblatts 8 ein die schlaufenbildende Komponente des Klettverschluss-Systems darstellendes flauschförmiges Frontaltape 23 vorgesehen. Die Verschlusselemente 22 und die Bandlasche 14 sind einstückig extrudiert. Verbindungsprobleme beim Zusammenfügen von Verschlusselementen an Haltelaschen treten daher nicht auf.

Wie aus Figur 2 zu ersehen ist, weist die Bandlasche 14 einen mittleren Abschnitt 24 und zwei sich daran anschließende Außenabschnitte 26 auf, wobei die Außenabschnitte 26 eine größere Materialdicke aufweisen, als der mittlere Abschnitt 24.

Die in Querrichtung 12 äußeren Kantenendbereiche 28 der Bandlasche 14 weisen keine Verschlusselemente 22 auf und bieten daher die Möglichkeit, den jeweiligen Kantenendbereich 28 mit den Fingern zu greifen, um das Verschluss-System zu öffnen.

In Figur 3 ist eine weitere Ausführungsform einer Bandlasche 14' dargestellt, bei der ein Teilbereich 30 der Außenabschnitte 26, der die mechanischen Verschlusselemente 22 trägt, eine noch größere dritte Materialdicke aufweist.

Schließlich ist aus Figur 4, die eine Draufsicht auf die Bandlasche 14' nach Figur 3 zeigt, ersichtlich, dass im mittleren Abschnitt 24 der Bandlasche 14' linear erstreckte Einschnitte 32 vorgesehen sind, die in Längsrichtung 18, also senkrecht zur Erstreckung der Bandlasche 14' verlaufen, und in Querrichtung 12 nebeneinander angeordnet sind. Die nebeneinander angeordneten Einschnitte 32 sind in Längsrichtung 18 versetzt zueinander. Hierdurch wird erreicht, dass bei im wesentlichen unelastischer Ausbildung der Bandlasche 14' in Verbindung mit einem elastischen Rückenblatt 8 eine gewisse Nachgiebigkeit der Bandlasche erreicht werden kann, indem die Einschnitte 32 beim Dehnen des Rückenblatts 8 rautenförmige Öffnungen bilden.

## Patentansprüche

1. Absorbierender Hygieneartikel zum einmaligen Gebrauch mit einem Rückenbereich (2) und einem Bauchbereich (4), umfassend ein zumindest abschnittsweise flüssigkeitsdurchlässiges Deckblatt (6), ein zumindest abschnittsweise flüssigkeitsdichtes Rückenblatt (8) und einen dazwischen angeordneten Saugkörper (10) sowie beidseits einen über einen seitlichen Längsrand (20) von Deckblatt (6) und Rückenblatt (8) überstehenden Abschnitt mit mechanisch wirkenden Verschlußelementen (22), die zum Verbinden von Rückenbereich (2) und Bauchbereich (4) im an den Körper eines Benutzers angelegten Zustand des Hygieneartikels mit Gegenverschlußelementen auf der Außenseite des Bauchbereichs (4) zusammenwirken und dabei diese Abschnitte mit den Verschlußelementen in Umfangsrichtung im Bauchbereich voneinander beabstandet sind, **dadurch gekennzeichnet, dass** im Rückenbereich (2) und in einer Querrichtung (12) des Hygieneartikels verlaufend eine einstückige, sich über die gesamte Breite (13) des Rückenbereichs (2) erstreckende, den Rückenbereich stabilisierende Bandlasche (14) angeordnet ist, die zumindest bereichsweise mit dem Rückenblatt (2) verbunden ist, und dass die Bandlasche (14) mit Endabschnitten (16) beidseits über den seitlichen Längsrand (20) übersteht und diese Endabschnitte (16) die Abschnitte mit den mechanisch wirkenden Verschlußelementen (22) bilden und dass die Bandlasche (14) im wesentlichen unelastisch ist oder zumindest eine geringere Elastizität aufweist als die des Rückenblatts (2).

2. Hygieneartikel nach Anspruch 1, ddg, dass die Bandlasche (14) zwischen dem Rückenblatt (8) und dem Deckblatt (6) angeordnet ist.

3. Hygieneartikel nach Anspruch 1, ddg, dass die mechanischen Verschlusselemente (22) Teil eines Klettverschluss-Systems sind.

4. Hygieneartikel nach Anspruch 2, ddg, dass die mechanischen Verschlusselemente (22) die hakenbildende Komponente eines Klettverschluss-Systems sind.

5. Hygieneartikel nach einem der vorstehenden Ansprüche, ddg, dass die Bandlasche (14) mitsamt der Verschlusselemente (22) in einem Stück extrudiert ist.

6. Hygieneartikel nach einem der vorstehenden Ansprüche, ddg, dass die Endabschnitte (16) der Bandlasche (14) den Kantenabschluss der Bandlasche bildende Kantenendbereiche (28) aufweisen, an denen keine mechanischen Verschlusselemente (22) vorgesehen sind und die somit als Greifbereich für die Finger eines Benutzers fungieren können.

7. Hygieneartikel nach einem der vorgenannten Ansprüche, ddg, die Bandlasche einen mittleren Abschnitt (24) mit einer ersten Materialstärke aufweist und sich beidseitig an den mittleren Abschnitt anschließende Außenabschnitte (26) mit einer zweiten Materialstärke, die größer ist als die erste Materialstärke, wobei die Endabschnitte (16) der Bandlasche (14) Teil der Außenabschnitte (26) der Bandlasche sind.

8. Hygieneartikel nach Anspruch 7, ddg, dass ein die mechanischen Verschlusselemente (22) tragender Teilbereich (32) der Außenabschnitte (26) eine dritte Materialstärke aufweist, die größer ist als die zweite Materialstärke.

9. Hygieneartikel nach einem der vorstehenden Ansprüche, **dadurch gekennzeichnet, dass** das zumindest abschnittsweise flüssigkeitsdichte Rückenblatt (8) atmungsaktiv ist.

10. Hygieneartikel nach Anspruch 9, ddg, dass das Rückenblatt (8) eine Folie umfasst.

11. Hygieneartikel nach Anspruch 10, ddg, dass die Folie Mikroporen mit einem Durchschnitt von ≤ 10 µm aufweist.

12. Hygieneartikel nach Anspruch 10 oder 11, ddg, dass die Folie Makroporen mit einer Projektionsfläche senkrecht zur Ebene der Folie von 0,1 bis 5 mm² aufweist.

13. Hygieneartikel nach einem der vorstehenden Ansprüche, ddg, dass das Rückenblatt (8) zumindest in Querrichtung elastisch ist.

14. Hygieneartikel nach einem der vorstehenden Ansprüche, ddg, dass die Bandlasche (14) im Bereich des Überlappens mit dem Rückenbereich (2) eine Vielzahl von die Bandlasche (14) durchdringenden Einschnitten aufweist.

15. Hygieneartikel nach Anspruch 14, **dadurch gekennzeichnet, dass** die Einschnitte im wesentlichen linear erstreckt sind.

16. Hygieneartikel nach Anspruch 14, **dadurch gekennzeichnet, dass** die Einschnitte in Längsrichtung des Hygieneartikels erstreckt sind und mehrere Einschnitte in Querrichtung nebeneinander und zur Längsrichtung versetzt zueinander angeordnet sind.

## Claims

1. Disposable absorbent sanitary article with a back region (2) and an abdominal region (4), comprising a cover sheet (6) which is liquid-permeable at least in portions, a backing sheet (8) which is liquid-tight at least in portions and an absorbent member (10) arranged therebetween and, on the two sides, a portion projecting beyond a lateral longitudinal edge (20) of cover sheet (6) and backing sheet (8) with mechanically acting sealing elements (22) which cooperate with counter-sealing elements on the outer side of the abdominal region (4) to connect the back region (2) and abdominal region (4) in the state of the sanitary article placed on the body of a wearer and in this process these portions with the sealing elements are spaced from one another in the peripheral direction in the abdominal region, **characterised in that** a one-piece tape strip (14) extending over the entire width (13) of the back region (2) and stabilising the back region is arranged to extend in the back region (2) and in a transverse direction (12) of the sanitary article, this tape strip (14) being connected at least at certain points to the backing sheet (2) and **in that** the tape strip (14) projects with end portions (16) on the two sides beyond the lateral longitudinal edge (20) and these end portions (16) form the portions with the mechanically acting sealing elements (22) and **in that** the tape strip (14) is substantially inelastic or at least has a lower elasticity than the backing sheet (2).

2. Sanitary article according to claim 1, **characterised in that** the tape strip (14) is arranged between the backing sheet (8) and the cover sheet (6).

3. Sanitary article according to claim 1, **characterised in that** the mechanical sealing elements (22) are part of a Velcro-type sealing system.

4. Sanitary article according to claim 2, **characterised in that** the mechanical sealing elements (22) are the hook-forming components of a Velcro-type sealing system.

5. Sanitary article according to any one of the preceding claims, **characterised in that** the tape strip (14) together with the sealing elements (22) is extruded in one piece.

6. Sanitary article according to any one of the preceding claims, **characterised in that** the end portions (16) of the tape strip (14) have the edge end regions (28) forming the edge closure of the tape strip, on which edge end regions (28) no mechanical sealing elements (22) are provided and which can therefore function as the gripping region for the fingers of a user.

7. Sanitary article according to any one of the preceding claims, **characterised in that** the tape strip has a central portion (24) with a first material thickness and outer portions (26) adjoining the central portion on the two sides with a second material thickness which is greater than the first material thickness, the end portions (16) of the tape strip (14) being part of the outer portions (26) of the tape strip.

8. Sanitary article according to claim 7, **characterised in that** a part region (32) of the outer portions (26) bearing the mechanical sealing elements (22) has a third material thickness which is greater than the second material thickness.

9. Sanitary article according to any one of the preceding claims, **characterised in that** the backing sheet (8) which is fluid-tight at least in portions is breathable.

10. Sanitary article according to claim 9, **characterised in that** the backing sheet (8) comprises a film.

11. Sanitary article according to claim 10, **characterised in that** the film has micropores with an average of ≤ 10 µm.

12. Sanitary article according to claim 10 or 11, **characterised in that** the film has macropores with a projection area perpendicular to the plane of the film of 0.1 to 5 mm².

13. Sanitary article according to any one of the preceding claims, **characterised in that** the backing sheet (8) is elastic at least in the transverse direction.

14. Sanitary article according to any one of the preceding claims, **characterised in that** the tape strip (14) in the region of overlap with the back region (2) has a plurality of incisions penetrating the tape strip (14).

15. Sanitary article according to claim 14, **characterised in that** the incisions extend in a substantially linear manner.

16. Sanitary article according to claim 14, **characterised in that** the incisions extend in the longitudinal direction of the sanitary article and a plurality of incisions are arranged next to one another in the transverse direction and offset from one another with respect to the longitudinal direction.

## Revendications

1. Article d'hygiène absorbant à usage unique comportant une zone dorsale (2) et une zone ventrale (4), comprenant une feuille de couverture (6) perméable aux liquides au moins par endroits, une feuille arrière (8) imperméable aux liquides au moins par endroits et un corps d'absorption (10) disposé entre les deux ainsi que, des deux côtés, une partie dépassant au-dessus d'un bord longitudinal (20) latéral de feuille de couverture (6) et de feuille arrière (8) ayant des éléments de fermeture (22) opérant mécaniquement, lesquels coopèrent avec des contre-éléments de fermeture situés sur la face extérieure de la zone ventrale (4) afin de relier la zone dorsale (2) et la zone ventrale (4) lorsque l'article d'hygiène est posé contre le corps d'un utilisateur, et ces parties avec les éléments de fermeture étant alors situées à distance l'une de l'autre dans la direction périphérique dans la zone ventrale, **caractérisé en ce qu**'une bande d'attache (14) d'un seul tenant, s'étendant sur toute la largeur (13) de la zone dorsale (2) et stabilisant la zone dorsale est disposée dans la zone dorsale (2) et dans une direction transversale (12) de l'article d'hygiène, laquelle bande d'attache (14) est reliée au moins par endroits à la feuille arrière (2), et en ce que la bande d'attache (14) dépasse avec des parties d'extrémité (16) des deux côtés au-dessus du bord longitudinal (20) latéral et ces parties d'extrémité (16) forment les parties avec les éléments de fermeture (22) opérant mécaniquement, et en ce que la bande d'attache (14) est essentiellement non élastique ou présente au moins une élasticité plus faible que celle de la feuille arrière (2).

2. Article d'hygiène selon la revendication 1, **caractérisé en ce que** la bande d'attache (14) est disposée entre la feuille arrière (8) et la feuille de couverture (6).

3. Article d'hygiène selon la revendication 1, **caractérisé en ce que** les éléments de fermeture mécaniques (22) font partie d'un système de fermeture auto-agrippant.

4. Article d'hygiène selon la revendication 2, **caractérisé en ce que** les éléments de fermeture mécaniques (22) sont les composants formant crochets d'un système de fermeture auto-agrippant.

5. Article d'hygiène selon l'une des revendications précédentes, **caractérisé en ce que** la bande d'attache (14) est extrudée d'une seule pièce avec les éléments de fermeture (22).

6. Article d'hygiène selon l'une des revendications précédentes, **caractérisé en ce que** les parties d'extrémité (16) de la bande d'attache (14) présentent des zones d'extrémité de bord (28) formant la terminaison de bord de la bande d'attache, sur lesquelles aucun élément de fermeture mécanique (22) n'est prévu et lesquelles peuvent ainsi faire office de zone de prise pour les doigts d'un utilisateur.

7. Article d'hygiène selon l'une des revendications précédentes, **caractérisé en ce que** la bande d'attache présente une partie centrale (24) ayant une première épaisseur de matière et des parties extérieures (26) se raccordant des deux côtés à la partie centrale et ayant une deuxième épaisseur de matière qui est supérieure à la première épaisseur de matière, les parties d'extrémité (16) de la bande d'attache (14) faisant partie des parties extérieures (26) de la bande d'attache.

8. Article d'hygiène selon la revendication 7, **caractérisé en ce qu'**une zone partielle (32) des parties extérieures (26) portant les éléments de fermeture mécaniques (22) présente une troisième épaisseur de matière, laquelle est supérieure à la deuxième épaisseur de matière.

9. Article d'hygiène selon l'une des revendications précédentes, **caractérisé en ce que** la feuille arrière (8) imperméable aux liquides au moins par endroits est imper-respirante.

10. Article d'hygiène selon la revendication 9, **caractérisé en ce que** la feuille arrière (8) comprend un film.

11. Article d'hygiène selon la revendication 10, **caractérisé en ce que** le film présente des micropores ayant une section ≤ 10 µm.

12. Article d'hygiène selon la revendication 10 ou 11, **caractérisé en ce que** le film présente des macropores ayant une surface de projection verticalement au plan du film comprise entre 0,1 et 5 mm².

13. Article d'hygiène selon l'une des revendications précédentes, **caractérisé en ce que** la feuille arrière (8) est élastique au moins dans la direction transversale.

14. Article d'hygiène selon l'une des revendications précédentes, **caractérisé en ce que** la bande d'attache (14) présente dans la zone du recouvrement avec la zone dorsale (2) une multiplicité d'incisions traversant la bande d'attache (14).

15. Article d'hygiène selon la revendication 14, **caractérisé en ce que** les incisions s'étendent essentiellement de manière linéaire.

16. Article d'hygiène selon la revendication 14, **caractérisé en ce que** les incisions s'étendent dans la direction longitudinale de l'article d'hygiène et plusieurs incisions sont disposées les unes à côté des autres dans la direction transversale et décalées les unes par rapport aux autres dans la direction longitudinale.
